Europäisches Patentamt

European Patent Office

Office européen des brevets

(11) Veröffentlichungsnummer: **0 295 432**
**A2**

# EUROPÄISCHE PATENTANMELDUNG

(21) Anmeldenummer: **88107701.0**

(22) Anmeldetag: **13.05.88**

(51) Int. Cl.⁴: **C12N 5/00 , C12N 9/50 , C12N 9/99**

Patentansprüche für folgende Vertragsstaaten:
ES + GR.

Die Bezeichnung der Erfindung wurde geändert
(Richtlinien für die Prüfung im EPA, A-III, 7.3).

(30) Priorität: **21.05.87 DE 3717029**

(43) Veröffentlichungstag der Anmeldung:
**21.12.88 Patentblatt 88/51**

(84) Benannte Vertragsstaaten:
**AT BE CH DE ES FR GB GR IT LI LU NL SE**

(71) Anmelder: **BEHRINGWERKE
Aktiengesellschaft
Postfach 1140
D-3550 Marburg 1(DE)**

(72) Erfinder: **Pâques, Eric Paul, Dr.
Schmiedeacker 18
D-3550 Marburg(DE)**

(74) Vertreter: **Meyer-Dulheuer, Karl-Hermann, Dr.
et al
HOECHST Aktiengesellschaft Zentrale
Patentabteilung Postfach 80 03 20
D-6230 Frankfurt/Main 80(DE)**

(54) **Animales Serum mit verringerter Protease- und Inhibitoraktivität.**

(57) Der Zusatz von animalen Seren zu Zellkulturen wirkt sich negativ auf die Aktivität der zu isolierenden Zellproteine aus. Die Erfindung betrifft somit ein Verfahren zum Behandeln eines animalen Serums, wobei das Serum mit wenigstens einem nukleophilen Agenz behandelt wird. Weiterhin betrifft die Erfindung ein nach dem beanspruchten Verfahren behandeltes Serum und die Verwendung dieses Serums als Zusatz zu einem Kulturmedium für Zellkulturen.

Die negativen proteolytischen und inhibitorischen Eigenschaften der verwendeten Seren werden selektiv vermindert, so daß die Aktivität der zu isolierenden Proteine bei Verwendung der erfindungsgemäßen Seren in hohem Maße erhalten bleibt.

EP 0 295 432 A2

## Verfahren zum Herstellen eines animalen Serums mit verringerter Protease- und Inhibitoraktivität, animales Serum, sowie Verwendung dieses Serums als Zusatz zu Kulturmedien

Die Erfindung betrifft ein Verfahren zur Behandlung eines animalen Serums, ein entsprechend diesem Verfahren behandeltes, animales Serum und die Verwendung eines solchen animalen Serums als Zusatz zu einem Kulturmedium für Zellkulturen.

Für die gezielte Produktion von Proteinen, die z.B. im Organismus natürlicherweise nur in äußerst geringen Mengen vorhanden sind, bedient man sich in zunehmendem Maß artifizieller, gleichwohl biologischer Systeme. In Abhängigkeit von der Natur des gewünschten Proteins besteht die Wahl des Wirtssystems, in dem die Proteine produziert werden sollen, zwischen grampositiven oder gramnegativen prokaryontischen Wirtssystemen, relativ einfachen Eukaryonten, wie z.B. Hefe, oder aber komplizierteren, technisch aufwendigeren Systemen, wie Oozyten oder Animalzellkulturen. Die Synthese von eukaryontischen, in vivo posttranslational modifizierten Proteinen wird in aller Regel nur bei Expression in eukaryontischen Zellen zu einem, dem in vivo Translationsprodukt vergleichbaren, Protein führen. Aus diesem Grund kommt der Produktion von Proteinen in Zellkulturen immer größere Bedeutung zu.

Zur Kultivierung animaler Zellen werden üblicherweise synthetische Medien verwendet, die durch einen geringen Zusatz animaler Seren supplementiert werden. Durch diesen Zusatz animalen Serums werden dem Medium zum einen wichtige, wachstumsfördernde Komponenten, wie z.B. Wachstumsfaktoren und Hormone, zum anderen aber auch unerwünschte Proteine, wie z.B. Proteinasen, oder Inhibitoren der gewünschten Proteine, zugefügt. Die Anwesenheit von Proteinasen erniedrigt die Ausbeute sezernierter Proteine unter Umständen drastisch und führt damit zu hohen Ausbeuteverlusten, während die Anwesenheit von Inhibitoren unter Umständen zur Inaktivierung der gewünschten Proteine führt.

Zur Vermeidung des proteolytischen Abbaus sezernierter Proteine wurden bisher Proteinaseinhibitoren wie z.B. Benzamidin, Orthophenanthrolin oder EDTA eingesetzt. Diese Inhibitoren können zum Teil nur in äußerst geringen Konzentrationen eingesetzt werden, da sie, wie Benzamidin und Orthophenanthrolin, - schlecht wasserlöslich sind, oder aber sie hemmen jeweils nur einzelne Proteaseklassen. So wird Benzamidin zur Hemmung von Serinproteasen eingesetzt, während z.B. EDTA auf $Zn^{2+}$-abhängige Enzyme wirkt. Ein weiterer Nachteil ist die Reversibilität der Inhibition, die z.B. nach Verdünnungsschritten erneutes Zufügen des Inhibitors erfordert.

Aufgabe der vorliegenden Erfindung war es deshalb, ein Verfahren zur Behandlung eines Animalserums zu entwickeln, durch das die unerwünschten, proteolytischen und inhibitorischen Eigenschaften dieses Serums verringert oder gar beseitigt werden.

Diese Aufgabe wird erfindungsgemäß dadurch gelöst, daß das Serum mit wenigstens einem nukleophilen Agenz behandelt wird.

Es hat sich überraschenderweise gezeigt, daß die Behandlung eines animalen Serums mit nukleophilen Agenzien die proteolytischen und inhibitorischen Eigenschaften des Serums verringert, ohne jedoch die wachstumsfördernden Eigenschaften zu beeinflussen. Dies konnte bei spielsweise durch den Vergleich der Aktivität verschiedener Proteine nach mehrstündiger Inkubation im Kulturmedium mit unbehandelten Serum einerseits sowie erfindungsgemäß behandeltem Serum andererseits nachgewiesen werden. Aus dem Vergleich geht eindeutig hervor, daß die Stabilität mehrerer untersuchter Proteine, gemessen als ihre Aktivität, in Kulturmedien mit Zusätzen erfindungsgemäß behandelter Seren deutlich erhöht war.

Für die Behandlung mit einem nukleophilen Agenz werden zweckmäßigerweise Amine eingesetzt.

Besonders bevorzugt ist die Verwendung von einem oder mehreren Aminen aus der Gruppe Methylamin, Hydrazin, Ethanolamin, Isopropylamin, Hydroxylamin, Propylamin, Butylamin, vorzugsweise in einer Konzentration von 0,001 mol/l bis 1 mol/l, besonders bevorzugt 0,05 mol/l bis 0,5 mol/l, insbesondere bevorzugt 0,1 mol/l bis 0,3 mol/l.

Ein bevorzugtes Agenz ist Ammoniak, das in einer Konzentration von 0,0005 bis 0,1 mol/l, besonders bevorzugt in einer Konzentration zwischen 0,001 bis 0,03 mol/l verwendet wird.

Als nukleophiles Agenz zur Behandlung des Serums kann weiterhin Phenol verwendet werden, vorzugsweise in einer Konzentration von 0,001 bis 1 mol/l, besonders bevorzugt 0,01 bis 0,1 mol/l, insbesondere bevorzugt in einer Konzentration von 0,05 mol/l.

Die Verwendung konzentrierten Phenols zur Denaturierung von Proteinen ist bereits bekannt; dieser Effekt wird vielfach, z.B. bei der Reinigung von Nukleinsäuren, ausgenutzt. Außerdem sind Phenolderivate in höheren Konzentrationen (1 bis 3 Prozent) als Desinfektionsmittel verwendet worden. Der hier beschriebene Effekt einer selektiven Inaktivierung von proteolytischen und inhibitorischen Aktivitäten durch Phenollösung ist äußerst überraschend.

Eine weitere Behandlungsmöglichkeit des Serums mit nukleophilen Agenzien besteht in der Verwendung von thiolhaltigen Substanzen wie Mercaptoethanol und/oder Dithiothreitol, vorzugsweise in einer Konzentration von 0,001 bis 1 mol/l, besonders bevorzugt 0,01 mol/l.

Die Verwendung dieser beiden Substanzen führt zur Inaktivierung der proteolytischen und inhibitorischen Aktivitäten, während die Funktion der Wachstumsfaktoren offenbar nicht beeinträchtigt wird.

Zweckmäßigerweise wird die Behandlung mit dem nukleophilen Agenz bei einem pH-Wert von 8 bis 11, bevorzugt bei pH 9 bis 10, durchgeführt.

Die Temperatur, bei der die Behandlung mit den nukleophilen Agenzien erfolgt, kann zwischen 4°C und 90°C liegen, bevorzugt aber 15°C bis 70°C betragen, besonders bevorzugt ist der Bereich zwischen 30°C und 60°C, der den Temperaturen, bei denen die Komponenten des Serums normalerweise wirksam sind, am nächsten kommt.

Die Behandlung sollte für 5 bis 2000 Minuten, vorzugsweise 30 bis 1000 Minuten, besonders bevorzugt 30 bis 360 Minuten, durchgeführt werden.

Im Anschluß an die Behandlung mit dem nukleophilen Agenz wird dieses entweder durch Dialyse gegen ein geeignetes Puffersystem, z.B. PBS, entfernt, oder aber im Fall der Behandlung mit Ammoniak, durch Zugabe geeigneter Chemikalien neutralisiert.

Durch Kontrollexperimente konnte gezeigt werden, daß der Effekt der Inaktivierung der proteolytischen Enzyme im Serum durch eine Säurebehandlung des Serums zusätzlich zu der Behandlung mit nukleophilen Agenzien verstärkt werden konnte. Die Säurebehandlung wird bevorzugt vor der Behandlung mit dem nukleophilen Agenz durchgeführt, vorzugsweise bei pH 3 bis 6, besonders bevorzugt bei pH 3,5 bis 5,5. Das Serum wird nach der Säurebehandlung mit NaOH auf den für die Behandlung mit dem nukleophilen Agenz gewünschten pH eingestellt.

Zweckmäßigerweise wird die Säurebehandlung bei einer Temperatur von 4°C bis 90°C, bevorzugt bei 15°C bis 60°C, durchgeführt, und zwar für 5 bis 2000 Minuten, bevorzugt 30 bis 300 Minuten.

Das so gewonnene Serum wird durch Sterilfiltration sterilisiert.

Das durch das beschriebene Verfahren gewonnene Serum erfüllt die eingangs genannten Forderungen, d.h., der Vergleich dieses Serums mit einem unbehandelten Serum zeigt eine eindeutige Verringerung proteolytischer Aktivitäten bei nahezu vollständigem Erhalt wachstumsfördernder Eigenschaften. So zeigt der Vergleich der Zelldichte von Zellkulturen in Medien mit Zusatz von unbehandeltem Serum einerseits und erfindungsgemäß behandelter Seren andererseits nach 88 Stunden Inkubation eine um nur 10 bis 25 Prozent verringerte Zelldichte, während die t-PA-Aktivität im Überstand der Zellkultur mit erfindungsgemäß behandeltem Serum um 50 bis 70 Prozent über dem Vergleichswert liegt. Darüberhinaus zeigt der Vergleich der Aktivitäten verschiedener Proteine, z.B. Urokinase, Protein-C, Thromboplastininhibitor und Faktor VIII, nach mehrstündiger Inkubation in Kulturmedien mit unbehandeltem Serum einerseits und erfindungsgemäß behandelten Seren andererseits, für die im Kulturmedium mit erfindungsgemäß behandelten Serenzusätzen inkubierten Proteine eine deutlich höhere, erhaltene Aktivität. Im Fall von Urokinase läßt sich auch nach 17 Std. Inkubation bei 37°C bei Verwendung eines erfindungsgemäß behandelten Serums keinerlei Aktivitätsverlust beobachten.

Das erfindungsgemäße Serum eignet sich insbesondere als Bestandteil eines Kulturmediums für Zellkulturen. Es kann als Zusatz für die Formulierung von Kulturmedien für alle bekannten Mammalienzellen verwendet werden, insbesondere für die Kultur von Fibroblasten, Monozyten, Epithelzellen, Endothelzellen, Lymphoblasten, sowie von transformierten, permanenten Mammalienzellen oder Hybridomazellen.

Das bevorzugte Anwendungsgebiet des erfindungsgemäßen Serums ist die Kultivierung von Protein produzierenden Zellen, insbesondere von Plasminogen-Aktivator produzierenden Zellen.

Die Erfindung wird im Folgenden, insbesondere durch die Beispiele, näher erläutert.

## Herstellen verschiedener Seren

Rinderserum (100 ml) wurde folgendermaßen behandelt:

1. Serum 1: unbehandelt

2. Serum 2: Der pH-Wert wurde durch Zugabe von 1 N HCl auf 3,5 eingestellt und das Serum 30 min. bei Raumtemperatur inkubiert. Anschließend wurde das Serum mittels Zugabe von NaOH auf pH 9,0 eingestellt, mit 200 mM Methylamin versetzt, 30 min. bei 56°C inkubiert, anschließend gegen PBS (phosphate-buffered saline) dialysiert und sterilfiltriert.

3. Serum 3: Serum 3 wurde analog zu Serum 2 behandelt. Jedoch wurde statt Methylamin 200 mM Hydroxylamin verwendet.

4. Serum 4: Der pH-Wert des Serums wurde durch Zugabe von 1 N HCl auf 3, 5 eingestellt und das Serum 30 min. bei Raumtemperatur inkubiert. Anschließend wurde das Serum durch Zugabe von Ammoniak auf pH 9,0 eingestellt, 30 min. bei 56°C inkubiert, mit Dihydrogencarbonat neutralisiert und sterilfiltriert.

5. Serum 5: Serum 5 wurde analog zu Serum 4 behandelt, jedoch ohne Inkubation mit Säure.

6. Serum 6: Das Serum wurde analog zu Serum 2 behandelt. Das Serum wurde jedoch mit 50 mM Phenol anstatt von Methylamin, 30 min. bei 56°C inkubiert.

7. Serum 7: Das Serum wurde analog zu Serum 2 behandelt. Das Serum wurde jedoch mit 10 mM Dithiothreitol anstatt von Methylamin versetzt und 30 min. bei Raumtemperatur inkubiert.

## Beispiel 1

t-PA wurde ähnlich wie in JBC 256 (13), 7035-7041 (1981) isoliert und anschließend mit "Dulbecco's Modified Eagle's Medium", enthaltend 5 % Rinderserum, auf 10.000 IU/ml verdünnt. Die t-PA-Aktivität wurde direkt nach Verdünnung sowie nach 6 Stunden Inkubation bei 37°C gemessen. Der Einfluß des Serums auf die t-PA-Aktivität wurde ermittelt.

| | Aktivität direkt nach Verdünnung | Aktivität nach 6 Stunden Inkubation bei 37°C |
|---|---|---|
| Medium ohne Serum | 100 % | 100 % |
| Medium mit Serum 1 | 100 % | 51 % |
| Medium mit Serum 2 | 100 % | 91 % |
| Medium mit Serum 3 | 100 % | 87 % |
| Medium mit Serum 4 | 100 % | 94 % |
| Medium mit Serum 5 | 100 % | 83 % |
| Medium mit Serum 6 | 100 % | 95 % |
| Medium mit Serum 7 | 100 % | 83 % |

Der Vergleich der verschiedenen Medien zeigt deutlich, daß die starke Aktivitätsverminderung im Serum 1 durch die Behandlung mit nukleophilen Agenzien, insbesondere aber durch eine zusätzliche Behandlung mit Säure, größtenteils aufgehoben werden kann.

**Beispiel 2:** Einfluß der Serum-Behandlung auf die t-PA-Aktivität im Zellkultur-Überstand.

t-PA-produzierende CHO-Zellen (Chinese Hamster Ovary) wurden im 4 ml Dulbecco's Modified Eagle's Medium, enthaltend 5 % Rinderserum Nr.1, 4 bzw. 5 (Beispiel 1) in Petrischalen (6 cm Ø ) für Gewebekultur gezüchtet. Die Kulturen wurden mit einer Zelldichte von $5 \times 10^4$ Zellen/ml angesetzt. Nach 64 Stunden wurde das Medium gewechselt. Nach 88 Stunden wurde die Zelldichte sowie auch die t-PA-Aktivität im Zellkultur-Überstand nach der Methode von Rånby (Progress in Fibrinolysis 5, 233-235 (1981)-)bestimmt. Die Zellkulturüberstände wurden geerntet und 4 Stunden bei 37°C inkubiert. Die t-PA-Aktivität wurde anschließend ermittelt.

Aus Tabelle 1 ist zu entnehmen, daß die t-PA-Aktivität ($IU/10^6$ Zellen/Tag) in den Kulturüberständen, enthaltend die erfindungsgemäß behandelten Seren 4 und 5, deutlich höher lag, als in dem Kontrollversuch. Weiterhin wurde beobachtet, daß die t-PA-Aktivität in den Medien, enthaltend die Seren 4 bzw. 5, nach 4 Stunden Inkubation bei 37°C deutlich stabiler bleibt, als im Kontrollversuch.

## Tabelle 1

|  | Zelldichte nach 88 Std. ($\times 10^5$ Zellen/ml) | t-PA-Aktivität im Zellkultur-überstand ($IU/10^6$ Zellen/Tag) | t-PA-Aktivität nach 4 Std. Inkuba. des Zellkulturüber-standes bei 37°C ($IU/10^6$ Zellen/Tag) |
|---|---|---|---|
| Kontrolle (Serum 1) | 4.9 / 3.2 | 1148 / 1489 | 749 / 818 |
| Serum 4 | 3.5 / 2.5 | 1621 / 2334 | 1521 / 1899 |
| Serum 5 | 4.6 / 2.8 | 1713 / 2703 | 1600 / 2483 |

Der Vergleich der t-PA-Aktivität in den Zellkulturüberständen zeigt für die Kulturmedien mit erfindungsgemäß behandelten Serenzusätzen eine um 50 bis 70% erhöhte t-PA-Aktivität. Nach vierstündiger Inkubation des Zellkulturüberstandes erhöht sich der Unterschied auf 120 bis 170 %.

**Beispiel 3:** Einfluß der Serum-Behandlung auf die Stabilität verschiedener Proteine.

Die Stabilität verschiedener Proteine in "Dulbecco's modified Eagle's Medium", enthaltend 5 % des Serums 1 und des Serums 4, wurde untersucht. Die Proteinlösungen wurden mit den Medien 1 : 10 verdünnt und die Aktivität anschließend sowie auch nach 4 bzw. 17 Stunden Inkubation bei 37° C getestet. Es wurde der Einfluß der Serum-Behandlung auf die Stabilität folgender Proteine untersucht:

a) Urokinase: Urokinase wurde, wie in DE 34 39 980 beschrieben, aus Urin isoliert. Die Urokinase-Konzentration der Lösung betrug 1.000 IU/ml.

b) Human-Protein C wurde, wie in Thromb.Haemost. 48 1 (1982) von Bertina beschrieben, isoliert. Die Protein-C-Konzentration der Lösung betrug 200 µg/ml.

c) Thromboplastin-Inhibitor wurde wie von Y.Shidara in Acta Obst.Gynaec Jpn 36, (12), 2583-2592 (1984) veröffentlicht, aus Human-Plazenta isoliert. Die Thromboplastin-Inhibitor-Konzentration betrug 280 µg/ml.

d) F VIII: Hierfür wurde das Präparat der Behringwerke (Haemate ®) eingesetzt. Die F VIII-Konzentration der eingesetzten Lösung betrug 4 E/ml.

## Tabelle 2

| Protein | Medium | Inkubations-zeit | Aktivität sofort nach Verdünnung | Aktivität nach Inkubation bei 37°C |
|---|---|---|---|---|
| Urokinase | Medium + Serum 1 | 17 Std. | 100 % | 72.3 % |
|  | Medium + Serum 4 |  | 100 % | 100 % |
| Protein C | Medium + Serum 1 | 14 Std. | 100 % | 69 % |
|  | Medium + Serum 4 |  | 100 % | 80 % |
| Thrombo-plastin-Inhibitor | Medium + Serum 1 | 17 Std. | 100 % | 10.3 % |
|  | Medium + Serum 4 |  | 100 % | 60.4 % |
| F VIII | Medium + Serum 1 | 17 Std. | 100 % | 26.9 % |
|  | Medium + Serum 4 |  | 100 % | 45.5 % |

Aus der Tabelle geht eindeutig hervor, daß Aktivität der untersuchten Proteine bei Verwendung konventioneller Seren als Mediumzusatz zum Teil drastisch abnimmt, während die Aktivität der untersuchten Proteine in Medien mit Seren, die erfindungsgemäß behandelt worden sind, gar nicht oder deutlich weniger abgenommen hat.

Die Beispiele belegen, daß die Isolierung sezernierter Proteine in aktiver Form durch die Verwendung erfindungsgemäß behandelter Seren effizienter gestaltet werden kann. Dies ist einmal im Hinblick auf die eventuelle therapeutische Anwendung der so produzierten Proteine bedeutsam, da für viele Anwendungsformen hohe spezifische Aktivitäten des isolierten Produktes erforderlich sind. Darüberhinaus ist die Isolierung erhöhter Mengen Protein pro Volumeneinheit serumhaltigen Kulturmediums ein wirtschaftlich bedeutender Aspekt.


### Ansprüche

1. Verfahren zur Behandlung eines animalen Serums, dadurch **gekennzeichnet**, daß das Serum mit wenigstens einem nukleophilen Agenz behandelt wird.

2. Verfahren nach Anspruch 1, dadurch **gekennzeichnet**, daß die nukleophilen Agenzien Amine sind.

3. Verfahren nach Anspruch 2, dadurch **gekennzeichnet**, daß das Serum mit wenigstens einem Amin, ausgewählt aus der Gruppe Methylamin, Hydrazin, Ethanolamin, Isopropylamin, Hydroxylamin Propylamin, Butylamin, bevorzugt Hydroxylamin und/oder Methylamin, vorzugsweise in einer Konzentration von 0,001 bis 1 mol/l, besonders bevorzugt 0,05 bis 0,5 mol/l, insbesondere bevorzugt 0,1 bis 0,3 mol/l, behandelt wird.

4. Verfahren nach Anspruch 1, dadurch **gekennzeichnet**, daß das Serum mit Ammoniak, vorzugsweise in einer Konzentration von 0,0005 bis 0,1 mol/l, besonders bevorzugt 0,001 bis 0,03 mol/l, behandelt wird.

5. Verfahren nach Anspruch 1, dadurch **gekennzeichnet**, daß das Serum mit Phenol, vorzugsweise in einer Konzentration von 0,001 bis 1 mol/l, besonders bevorzugt 0,01 bis 0,1 mol/l, insbesondere bevorzugt 0,05 mol/l, behandelt wird.

6. Verfahren nach Anspruch 1, dadurch **gekennzeichnet**, daß das Serum mit Mercaptoethanol und/oder Dithiothreitol, vorzugsweise in einer Konzentration von 0,001 bis 1 mol/l, besonders bevorzugt 0,01 mol/l, behandelt wird.

7. Verfahren nach mindestens einem der Ansprüche 1 bis 6, dadurch **gekennzeichnet**, daß die Behandlung bei einem pH-Wert von 5 bis 11, bevorzugt bei pH 8 bis 11, besonders bevorzugt bei pH 9 bis 10, erfolgt.

8. Verfahren nach mindestens einem der Ansprüche 1 bis 7, dadurch **gekennzeichnet**, daß die Behandlung bei $4^\circ$ C bis $90^\circ$ C, bevorzugt bei $15^\circ$ C bis $70^\circ$ C, besonders bevorzugt bei $30^\circ$ C bis $60^\circ$ C, durchgeführt wird.

9. Verfahren nach mindestens einem der Ansprüche 1 bis 8, dadurch **gekennzeichnet**, daß die Behandlung 5 bis 2000 Minuten, vorzugsweise 30 bis 1000 Minuten, besonders bevorzugt 30 bis 360 Minuten, durchgeführt wird.

10. Verfahren nach mindestens einem der Ansprüche 1 bis 9, dadurch **gekennzeichnet**, daß das Serum vor oder nach der Behandlung mit nukleophilen Agenzien zusätzlich mit Säure, vorzugsweise bei pH 3 bis 6, besonders bevorzugt bei pH 3,5 bis 5,5, behandelt wird.

11. Verfahren nach Anspruch 10, dadurch **gekennzeichnet**, daß die Säurebehandlung bei $4^\circ$ C bis $90^\circ$ C, bevorzugt bei $15^\circ$ C bis $60^\circ$ C, durchgeführt wird.

12. Verfahren nach einem der Ansprüche 10 oder 11, dadurch **gekennzeichnet**, daß die Säurebehandlung 5 bis 2000 Minuten, bevorzugt 30 bis 300 Minuten, durchgeführt wird.

13. Verfahren nach mindestens einem der Ansprüche 1 bis 12, dadurch **gekennzeichnet**, daß das Serum steril filtriert wird.

14. Animales Serum, dadurch **gekennzeichnet**, daß es nach einem Verfahren gemäß einem der Ansprüche 1 bis 13 behandelt worden ist.

15. Verwendung eines animalen Serums nach Anspruch 14 als Zusatz zu einem Kulturmedium für Zellkulturen.

16. Verwendung eines animalen Serums nach Anspruch 14 für die Kultur von normalen Mammalienzellen, insbesondere Fibroblasten, Monocyten, Epithelzellen, Endothelzellen und Lymphoblasten oder transformierten permanenten Mammalienzellen oder Hybridomazellen.

17. Verwendung eines animalen Serums nach Anspruch 14 für die Produktion von Plasmaproteinen, bevorzugt t-PA, wobei die Aktivität der genannten Proteine nahezu vollständig stabil erhalten bleibt.

Patentansprüche für folgende Vertragsstaaten: ES und GR

1. Verfahren zur Behandlung animalen Serums, dadurch **gekennzeichnet**, daß das Serum mit wenigstens einem nukleophilen Agenz behandelt wird.

2. Verfahren nach Anspruch 1, dadurch **gekennzeichnet**, daß die nukleophilen Agenzien Amine sind.

3. Verfahren nach Anspruch 2, dadurch **gekennzeichnet**, daß das Serum mit wenigstens einem Amin, ausgewählt aus der Gruppe Methylamin, Hydrazin, Ethanolamin, Isopropylamin, Hydroxylamin, Propylamin, Butylamin, bevorzugt Hydroxylamin und/oder Methylamin, vorzugsweise in einer Konzentration von 0,001 bis 1 mol/l, besonders bevorzugt 0,05 bis 0,5 mol/l, insbesondere bevorzugt 0,1 bis 0,3 mol/l, behandelt wird.

4. Verfahren nach Anspruch 1, dadurch **gekennzeichnet**, daß das Serum mit Ammoniak, vorzugsweise in einer Konzentration von 0,0005 bis 0,1 mol/l, besonders bevorzugt 0,001 bis 0,03 mol/l, behandelt wird.

5. Verfahren nach Anspruch 1, dadurch **gekennzeichnet**, daß das Serum mit Phenol, vorzugsweise in einer Konzentration von 0,001 bis 1 mol/l, besonders bevorzugt 0,01 bis 0,1 mol/l, insbesondere bevorzugt 0,05 mol/l, behandelt wird.

6. Verfahren nach Anspruch 1, dadurch **gekennzeichnet**, daß das Serum mit Mercaptoethanol und/oder Dithiothreitol, vorzugsweise in einer Konzentration von 0,001 bis 1 mol/l, besonders bevorzugt 0,01 mol/l, behandelt wird.

7. Verfahren nach mindestens einem der Ansprüche 1 bis 6, dadurch **gekennzeichnet**, daß die Behandlung bei einem pH-Wert von 5 bis 11, bevorzugt bei pH 8 bis 11, besonders bevorzugt bei pH 9 bis 10, erfolgt.

8. Verfahren nach mindestens einem der Ansprüche 1 bis 7, dadurch **gekennzeichnet**, daß die Behandlung bei 4°C bis 90°C, bevorzugt bei 15°C bis 70°C, besonders bevorzugt bei 30°C bis 60°C, durchgeführt wird.

9. Verfahren nach mindestens einem der Ansprüche 1 bis 8, dadurch **gekennzeichnet**, daß die Behandlung 5 bis 2000 Minuten, vorzugsweise 30 bis 1000 Minuten, besonders bevorzugt 30 bis 360 Minuten, durchgeführt wird.

10. Verfahren nach mindestens einem der Ansprüche 1 bis 9, dadurch **gekennzeichnet**, daß das Serum vor oder nach der Behandlung mit nukleophilen Agenzien zusätzlich mit Säure, vorzugsweise bei pH 3 bis 6, besonders bevorzugt bei pH 3,5 bis 5,5, behandelt wird.

11. Verfahren nach Anspruch 10, dadurch **gekennzeichnet**, daß die Säurebehandlung bei 4°C bis 90°C, bevorzugt bei 15°C bis 60°C, durchgeführt wird.

12. Verfahren nach einem der Ansprüche 10 oder 11, dadurch **gekennzeichnet**, daß die Säurebehandlung 5 bis 2000 Minuten, bevorzugt 30 bis 300 Minuten, durchgeführt wird.

13. Verfahren nach mindestens einem der Ansprüche 1 bis 12, dadurch **gekennzeichnet**, daß das Serum steril filtriert wird.

14. Verwendung eines animalen Serums nach Anspruch 1 als Zusatz zu einem Kulturmedium für Zellkulturen.

15. Verwendung eines animalen Serums nach Anspruch 1 für die Kultur von normalen Mammalienzellen, insbesondere Fibroblasten, Monocyten, Epithelzellen, Endothelzellen und Lymphoblasten oder transformierten permanenten Mammalienzellen oder Hybridomazellen.

16. Verwendung eines animalen Serums nach Anspruch 1 für die Produktion von Plasmaproteinen, bevorzugt t-PA, wobei die Aktivität der genannten Proteine nahezu vollständig stabil erhalten bleibt.